# EUROPEAN PATENT SPECIFICATION

(11) **EP 4 619 375 B1**
(45) Date of publication and mention of the grant of the patent: **30.09.2026**
(21) Application number: 23804957.1
(22) Date of filing: 06.11.2023
(51) Int. Cl.: C07C 63/26, C07C 51/43, C07C 51/47

(54) **METHOD OF PURIFICATION OF TEREPHTHALIC ACID**
VERFAHREN ZUR REINIGUNG VON TEREPHTHALSÄURE
PROCÉDÉ DE PURIFICATION D'ACIDE TÉRÉPHTALIQUE

(30) Priority: 16.11.2022 US 202263425771 P
(43) Date of publication of application: 24.09.2025
(73) Proprietor: DePoly SA, 1950 Sion (CH)
(72) Inventor: ANDERSON, Samantha, 1950 Sion, Valais (CH); IRELAND, Christopher, 1950 Sion, Valais (CH); HLAIEM, Mohamed, 1950 Sion, Valais (CH)
(74) Representative: Prins Intellectual Property AG
(86) International application number: PCT/EP2023/080894
(87) International publication number: WO 2024/104811

(56) References cited:
- WO-A1-2020/173961
- JP-A- 2001 151 709

## Description

### Technical Field

The following relates generally to methods of purifying terephthalic acid (TPA). Specifically, the following relates to a method of purifying terephthalic acid (TPA) at room temperature, including the application of graphite, activated carbon and molecular sieves.

### Introduction

PCT Application Publication No. WO2020173961A1 provides a method of alkaline hydrolysis of one or more plastic polymers into terephthalic acid (TPA) and/or ethylene glycol (EG) and/or other monomers that form the one or more plastic polymers, the method comprising:
a) contacting the one or more plastic polymers with a metal oxide in a solution in the presence of a base to provide a reaction mixture;
b) stirring the reaction mixture during appropriate time under UV light;
c) recovering terephthalic acid, ethylene glycol and/or the other monomers from the reaction mixture.

The methods of PCT Application Publication No. WO2020173961A1 may output terephthalic acid that is contaminated or otherwise of a quality that requires further purification before further processing and use. Current methods of purifying TPA are energy intensive, and economically inefficient.

Accordingly, there is a need for an improved method for purifying TPA.

### Summary

Described herein is a method of purifying terephthalic acid, according to an embodiment. The method includes contacting unpurified terephthalic acid with graphite, activated carbon, and molecular sieve to provide a reaction mixture, stirring the reaction mixture for a first certain period of time, filtering the reaction mixture, to provide a reaction mixture filtrate, providing graphite, activated carbon, and molecular sieve to the reaction mixture filtrate, stirring the reaction mixture filtrate for a second certain period of time, filtering the reaction mixture filtrate, to provide a reaction output solution and precipitating purified terephthalic acid from the reaction output solution.

According to some embodiments, the reaction mixture is stirred at a pH of 14.

According to some embodiments, the reaction mixture filtrate is stirred at a pH of 7.

According to some embodiments, graphite, activated carbon, and molecular sieve are provided for contacting with the unpurified terephthalic acid at a 1:6:2 ratio.

According to some embodiments, graphite, activated carbon, and molecular sieve are provided to the reaction mixture filtrate at a 1:6:2 ratio.

According to some embodiments, purified terephthalic acid is precipitated from the reaction output solution using an acid.

According to some embodiments, the acid is hydrochloric acid or sulfuric acid.

According to some embodiments, the first period of time is between 10 and 120 minutes.

According to some embodiments, the first period of time is 30 minutes.

According to some embodiments, the second period of time is between 10 and 120 minutes.

According to some embodiments, the second period of time is 30 minutes.

According to some embodiments, the method is performed at room temperature.

According to some embodiments, the molecular sieve contacted with the unpurified terephthalic acid is Zeolite 13X.

According to some embodiments, the molecular sieve provided to the reaction mixture filtrate is Zeolite 13X.

Other aspects and features will become apparent to those ordinarily skilled in the art, upon review of the following description of some exemplary embodiments.

### Brief Description of the Drawings

The drawings included herewith are for illustrating various examples of articles, methods, and apparatuses of the present specification. In the drawings:
Figure 1 is a flow chart of a method of purifying terephthalic acid, according to an embodiment;
Figure 2 is a table detailing chemical and physical properties of terephthalic acid produced using the method of Figure 1, according to an embodiment;
Figure 3 is a table detailing chemical and physical properties of terephthalic acid produced using the method of Figure 1, according to another embodiment; and
Figure 4 is a table detailing chemical and physical properties of terephthalic acid produced using the method of Figure 1, according to another embodiment.

### Detailed Description

Various apparatuses or processes will be described below to provide an example of each claimed embodiment. No embodiment described below limits any claimed embodiment and any claimed embodiment may cover processes or apparatuses that differ from those described below. The claimed embodiments are not limited to apparatuses or processes having all of the features of any one apparatus or process described below or to features common to multiple or all of the apparatuses described below.

Further, although process steps, method steps, algorithms or the like may be described (in the disclosure and / or in the claims) in a sequential order, such processes, methods and algorithms may be configured to work in alternate orders. In other words, any sequence or order of steps that may be described does not necessarily indicate a requirement that the steps be performed in that order. The steps of processes described herein may be performed in any order that is practical. Further, some steps may be performed simultaneously.

When a single device or article is described herein, it will be readily apparent that more than one device / article (whether or not they cooperate) may be used in place of a single device / article. Similarly, where more than one device or article is described herein (whether or not they cooperate), it will be readily apparent that a single device / article may be used in place of the more than one device or article.

Associated with the screw conveyor reactor system described herein is a method of degradation of plastic materials into terephthalic acid (TPA), ethylene glycol and/or other monomers that form the plastic materials.

The method comprises: contacting the one or more plastic polymers with a metal oxide in a solution in the presence of a base to provide a reaction mixture;
Stirring the reaction mixture for an appropriate time under UV light; and
Recovering terephthalic acid, ethylene glycol and/or the other monomers from the reaction mixture.

The process comprises an alkaline hydrolysis of polymers, namely polyethylene terephthalate (PET). This process may be conducted at room temperature, with relatively high efficiency, in comparison to other methods of polymer degradation into constituent monomers.

In some embodiments, the solvent is ethanol or an ethanol-water mixture.

In some embodiments, the polymer is polyethylene terephthalate (PET).

In some embodiments, the metal oxide is TiO₂.

In some embodiments, the base is NaOH.

In some embodiments, the reaction mixture has an initial pH of 14.

In some embodiments, the reaction mixture is stirred at room temperature.

After recovery of the terephthalic acid after the conclusion of this process, the terephthalic acid may be of low purity and may require further processing to obtain terephthalic acid that is commercially useful, wherein the terephthalic acid may be provided to processes configured to require virgin terephthalic acid.

Common methods of purifying TPA typically include recrystallization steps, which require heating to high temperatures (e.g. 200°C), which is energy intensive, and require the use of harmful solvents, such as dimethylformamide. Such solvents may risk operator safety and require expensive, time consuming and complicated procedures to handle and manage.

Described herein is a method of purifying TPA, and other compounds. While the systems and methods described herein may be particularly well suited for use for the purification of TPA produced by the room temperature alkaline polymer hydrolysis process described above, and in PCT Application Publication No. WO2020173961A1, in some embodiments, the TPA purification method described herein may be applied to TPA from other sources, as well as compounds other than TPA. By using three adsorption materials, each in significant excess, and applying two adsorption steps at different pH levels, the adsorption material removes a high proportion of the contaminants associated with the TPA derived from contaminated PET. The method described herein may reliably output a purified TPA which is up to 1% purer and 5% brighter white in color than virgin TPA. In embodiments wherein compounds other than TPA are being purified, additional pH adjustments may be performed.

The methods described herein may be adapted to purify other compounds, such as low molecular weight (<200g/mol) monomers, for example, lactic acid. In other examples, water, such as wastewater, air, proteins and other substances may be purified by the methods described herein.

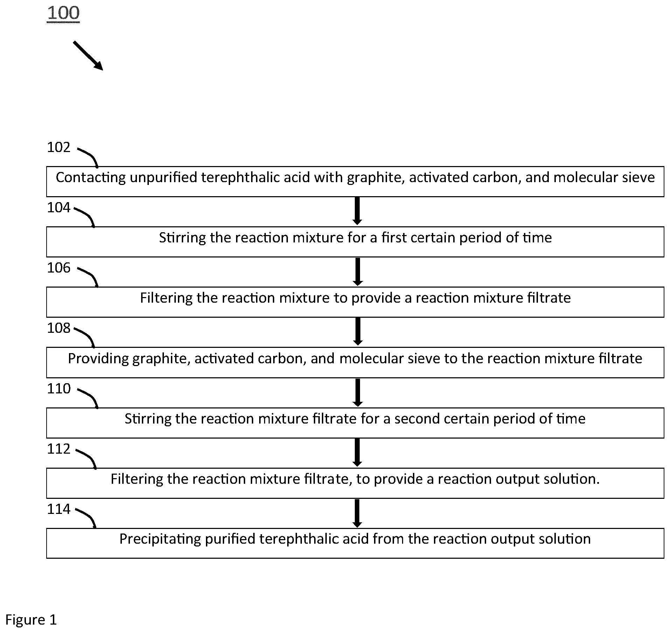

Referring now to Figure 1, pictured therein is a flow chart outlining a method 100 of purifying TPA, according to an embodiment. Method 100 comprises steps 102, 104, 106, 108, 110, 112, and 114.

The method described herein may be conducted as a batch operation, and performed in a reactor vessel, preferably equipped with an agitator, and a plug drain filter. In some examples, the method described herein may be conducted as a batch operation, across multiple reaction vessels, in a series configuration. In some examples, the method described herein may be modified to be operated as a continuous process.

At step 102, unpurified terephthalic acid is contacted with graphite, activated carbon, and molecular sieve. The unpurified terephthalic acid may be sourced from the output of a polymer recycling process, such as the room temperature alkaline polymer hydrolysis process described in PCT Application Publication No. WO2020173961A1. In some examples, the contacting processing of step 102 may be conducted within a reaction vessel.

In some examples, such unpurified terephthalic acid may comprise contaminants including dyes (such as azo dyes including Pigment Yellow 13, Sudan Black B, Sudan Red G, or anthraquinones such as Disperse Red 11), solid pigments such as carbon black, isostructural monomers such as phthalic acid, isophthalic acid, as well as molecules that can be present as contaminants in PET such as benzoic acid, p-toluic acid, bisphenol A, 4-carboxybenzaldehyde, metal ions such as chromium, iron, nickel, antimony, sodium, titanium, aluminum, barium, calcium, cobalt, manganese, molybdenum, lithium, potassium, zinc and/or other contaminants.

The graphite provided at step 102 may comprise amorphous, crystalline or flake graphite, of a purity of 99% and a particle size range of 5-30 µm (microns). In other examples, graphite of different purity levels, forms, and particle sizes may be alternatively provided.

In some examples, the graphite of step 102 may comprise a very fine back powder, with a crystallite height of 60 - 100 nm, an interlayer distance of 0.25 - 0.35 nm, a D90 particle size of 5 - 30 microns, a BET surface area of 10 - 15 m²/g, ash content less than 0.05%, and metal content generally less than 2ppm for individual metals.

The activated carbon provided at step 102 may comprise particulate form activated carbon with a surface area of 500-1500 m²/g, and a particle size less than 1mm.

In other examples, activated carbon of different purity levels, forms, surface areas, and particle sizes may be alternatively provided. In some examples, the activated carbon of step 102 may comprise a grinding fineness (less than 40 µm) of 70% by weight, an Iodine index of 900 - 1500 mg/g and a surface area of 800 - 1500 m²/g.

The molecular sieve provided at step 102 may comprise an aluminosilicate crystal molecular sieve, such as Zeolite 13X molecular sieve. Zeolite 13X comprises average pores measuring 9 angstrom, and may absorb molecules with a kinetic diameter smaller than 9 angstrom.

In some examples, the molecular sieve of step 102 may comprise 3 - 5 µm particles, with an average surface area of 700 m²/g. In other examples, other molecular sieves with similar compositions and/or specifications may be alternatively provided. In other examples, other molecular sieves with different compositions and/or specifications may be alternatively provided.

The graphite, activated carbon, and molecular sieve provided at step 102 may be provided at a 1:6:2 mass ratio of graphite, activated carbon, and molecular sieve respectively. In other examples, other ratios of graphite, activated carbon, and molecular sieve may be provided.

In some examples, the unpurified terephthalic acid may be in the form of M-TPA (with "M" denoting a metal such as Na⁺ or K⁺ that may originate from a hydrolysis process) dissolved in water at pH 14, at close to the maximum solubility of M-TPA in solution at ~ 13% by weight. In some examples, after step 102, 210L of total solution comprises 3 kg of activated carbon, 1 Kg of Zeolite 13X and 0.5 kg of graphite.

All three components added at step 102 allow capture of contaminants by an adsorption processes. Activated carbon may remove dyes by adsorbing the dye withing the large size of the material's internal pores. The relatively smaller pores of the Zeolite 13X compared to the activated carbon allows the capture of smaller molecular weight organic molecules. The surface of the graphite also allows nonchemical soft bonding of organic and metal ions. By altering the pH from basic to neutral, the surface charge of the absorbent material changes, from negative to neutral to positive, or positive to neutral to negative, allowing easier adsorption of organic molecules with a slightly negative or positive area of the molecule, in addition to heavy metals.

At step 104, the reaction mixture is stirred for a first certain period of time. The reaction mixture may be stirred within a reaction vessel, with an integrated agitator component, or by another similar device.

The reaction mixture may be stirred for a range of time between 10 and 120 minutes. In some examples, the reaction mixture may be stirred at step 104 for 60 minutes. In other examples, reaction mixture may be stirred for a different period of time.

At step 104, the reaction mixture may be stirred at an alkaline pH. pH adjusting compounds, such as HCl, NaOH, or another compound may be added to the reaction mixture before stirring to adjust the pH of the reaction mixture. In some examples, the reaction mixture may be stirred at a pH of 14.

At step 106, the reaction mixture is filtered, to provide a reaction mixture filtrate. In some examples, the reaction mixture may be filtered with a plug drain filter coupled to or integral to a reaction vessel at which steps 102 and/or 104 were performed. The filter may separate the solid components of the reaction mixture from liquid components. The filter may comprise a pore size slightly smaller than the diameter than the smallest solid component of the reaction mixture. For example, if the smallest solid particle comprises a diameter of 5 µm (microns), the filter may comprise a pore size of 4 µm.

After filtration, the reaction mixture filtrate may be provided to another vessel for further processing.

In some embodiments of the methods described herein, steps 102 - 106 may each be separated into multiple sub-steps performed in sequence. For example, only one of the graphite, activated carbon, and molecular sieve may be provided at step 102, and the mixture may then be subsequently stirred and filtered (as per steps 104 and 106). After filtration, another one of the graphite, activated carbon, and molecular sieve (for example, one which has not yet been provided in the method) may be provided and the mixture may then be subsequently stirred and filtered (as per steps 104 and 106). After the second filtration, another one of the graphite, activated carbon, and molecular sieve (for example, the final one which has not yet been provided in the method) may be provided and the mixture may then be subsequently stirred and filtered a final time (as per steps 104 and 106), producing the reaction mixture filtrate referred to at step 106.

In some examples, steps 102 - 106 may each be separated into two sub steps, wherein two of the graphite, activated carbon, and molecular sieve are provided in a first sub step, and the remaining material may be provided in a second sub step. In some examples, the second sub step may occur before the first sub step.

In some examples, steps 102 - 106 may each be separated into three sub steps, wherein one of the graphite, activated carbon, and molecular sieve are provided in a first sub step, one of the two remaining materials may be provided in a second sub step, and the third remaining material may be provided in a third sub step. In some examples, the first, second and third sub steps may be performed in any order.

In some examples, steps 102 - 106 may be performed using a stepwise column filtration system.

At step 108, graphite, activated carbon, and molecular sieve is provided to the reaction mixture filtrate. The graphite provided at step 108 may comprise amorphous, crystalline or flake graphite, of a purity of 99% and a particle size range of 5-30 µm (microns). In other examples, graphite of different purity levels, forms, and particle sizes may be alternatively provided.

In some examples, the graphite of step 108 may comprise a very fine back powder, with a crystallite height of 60 - 100 nm, an interlayer distance of 0.25 - 0.35 nm, a D90 particle size of 5 - 30 µm, a BET surface area of 10 - 15 m²/g, ash content less than 0.05%, and metal content generally less than 2ppm for individual metals.

The activated carbon provided at step 108 may comprise particulate form activated carbon with a surface area of 500-1500 m2/g, and a particle size less than 1 mm. In other examples, activated carbon of different purity levels, forms, surface areas, and particle sizes may be alternatively provided.

In some examples, the activated carbon of step 108 may comprise a grinding fineness (less than 40 µm) of 70% by weight, an Iodine index of 900 - 1500 mg/g and a surface area of 800 - 1500 m²/g.

The molecular sieve provided at step 108 may comprise an aluminosilicate crystal molecular sieve, such as Zeolite 13X molecular sieve. Zeolite 13X comprises average pores measuring 9 angstrom, and may absorb molecules with a kinetic diameter smaller than 9 angstrom.

In some examples, the molecular sieve of step 108 may comprise 3 - 5 µm particles, with an average surface area of 700 m²/g. In other examples, other molecular sieves with similar compositions and/or specifications may be alternatively provided. In other examples, other molecular sieves with different compositions and/or specifications may be alternatively provided.

The graphite, activated carbon, and molecular sieve provided at step 108 may be provided at a 1:6:2 mass ratio of graphite, activated carbon, and molecular sieve respectively. In other examples, other ratios of graphite, activated carbon, and molecular sieve may be provided, for example, as described above in reference to step 108.

At step 110, the reaction mixture filtrate is stirred for a second certain period of time. The reaction mixture filtrate may be stirred within a reaction vessel, with an integrated agitator component.

The reaction mixture filtrate may be stirred for a range of time between 10 and 120 minutes. In some examples, the reaction mixture filtrate may be stirred at step 110 for 30 minutes. In other examples, reaction mixture filtrate may be stirred for a different period of time.

At step 110, the reaction mixture filtrate may be stirred at a neutral pH. pH adjusting compounds, such as HCl, NaOH, or another similar compound may be added to the reaction mixture before stirring to adjust the pH of the reaction mixture filtrate. In some examples, the reaction mixture may be stirred at a pH of 7.

At step 112, the reaction mixture filtrate is filtered, to provide a reaction output solution. In some examples, the reaction mixture filtrate may be filtered with a plug drain filter coupled to, or integral to, a reaction vessel in which any one of steps 102 - 110 were performed. The filter may separate the solid components of the reaction mixture filtrate from liquid components. The filter may comprise a pore size slightly smaller than the diameter than the smallest solid component of the reaction mixture. For example, if the smallest solid particle (e.g. a particle of graphite) comprises a diameter of 5 µm , the filter may comprise a pore size of 4 µm.

Once the reaction mixture filtrate is filtered at step 112, the reaction output solution may be provided to another vessel, or back into the same vessel, for further processing.

In some embodiments of the methods described herein, steps 108 - 112 may each be separated into multiple sub-steps performed in sequence. For example, only one of the graphite, activated carbon, and molecular sieve may be provided at step 102, and the mixture may then be subsequently stirred and filtered (as per steps 110 and 112). After filtration, another one of the graphite, activated carbon, and molecular sieve (for example, one which has not yet been provided in the method) may be provided and the mixture may then be subsequently stirred and filtered (as per steps 110 and 11). After the second filtration, another one of the graphite, activated carbon, and molecular sieve (for example, the final one which has not yet been provided in the method) may be provided and the mixture may then be subsequently stirred and filtered a final time (as per steps 110 and 112), producing the reaction output solution referred to at step 112.

In some examples, steps 108 - 112 may each be separated into two sub steps, wherein two of the graphite, activated carbon, and molecular sieve are provided in a first sub step, and the remaining material may be provided in a second sub step. In some examples, the second sub step may occur before the first sub step.

In some examples, steps 108 - 112 may each be separated into three sub steps, wherein one of the graphite, activated carbon, and molecular sieve are provided in a first sub step, one of the two remaining materials may be provided in a second sub step, and the third remaining material may be provided in a third sub step. In some examples, the first, second and third sub steps may be performed in any order.

In some examples, steps 108 - 112 may be performed using a stepwise column filtration system.

At step 114, purified terephthalic acid is precipitated from the reaction output solution. In some examples, purified terephthalic acid may be precipitated by the addition of an acid to the reaction output solution. In some examples, this acid may comprise hydrochloric acid, or sulfuric acid. In other examples, any other suitable acid may be used to facilitate precipitation.

The acid provided at step 114 may have a concentration of 98% if sulfuric acid is provided. The acid provided at step 114 may have a concentration of 33% if hydrochloric acid is provided. Other suitable acids if provided will have a comparable concentration, ranging from 1-98%.

Once the purified terephthalic acid is precipitated from the reaction output solution, the purified terephthalic acid may separated from the solution by a means (e.g. filtration). In some examples, the purified terephthalic acid may be subject to additional processing steps (e.g. heating, vacuum drying, or other processing steps). For example, the purified terephthalic acid may be subjected to a grinding step, to produce a purified terephthalic acid in free-flowing powder form. Such a free-flowing powder may be transferred to a storage medium, and vacuum sealed, or stored in some other manner.

Once extracted through the application of method 100, the purified terephthalic acid may be applied to subsequent chemical processes and applications for which virgin quality terephthalic acid is required.

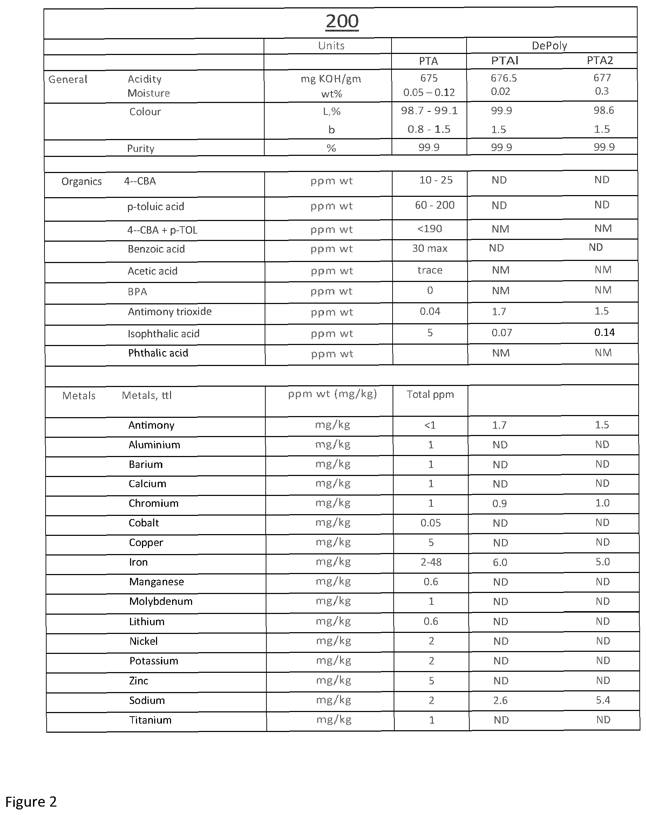

Referring now to Figure 2, shown therein is a table 200 detailing specifications of a purified terephthalic acid produced using method 100 described herein. Specifications detailed in table 200 include the concentration of contaminants and properties of two samples of purified terephthalic acid. The samples may be compared to PTA standards, as shown in Figure 2.

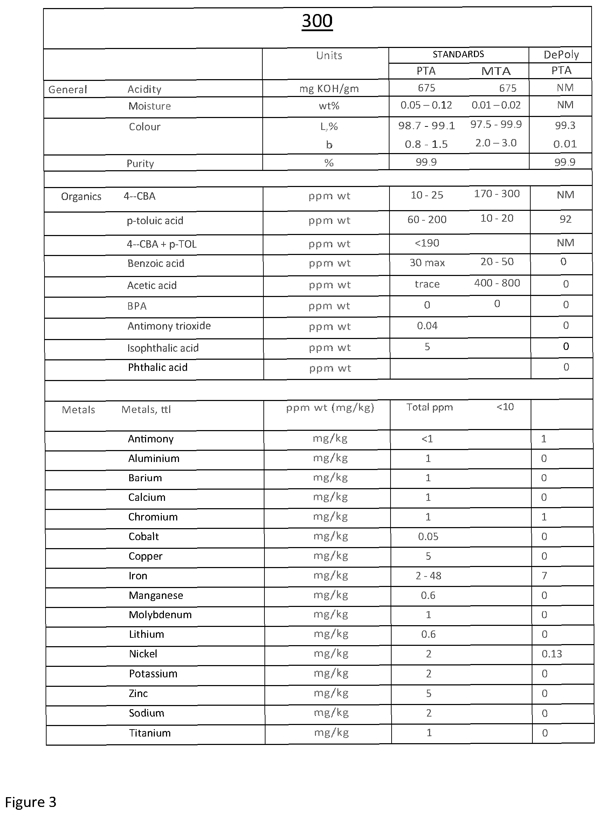

Referring now to Figure 3, shown therein is a table 300 detailing specifications of a purified terephthalic acid produced using method 100 described herein. Specifications detailed in table 300 include the concentration of contaminants in a sample of purified terephthalic acid. The sample may be compared to the listed standards, as shown in Figure 3.

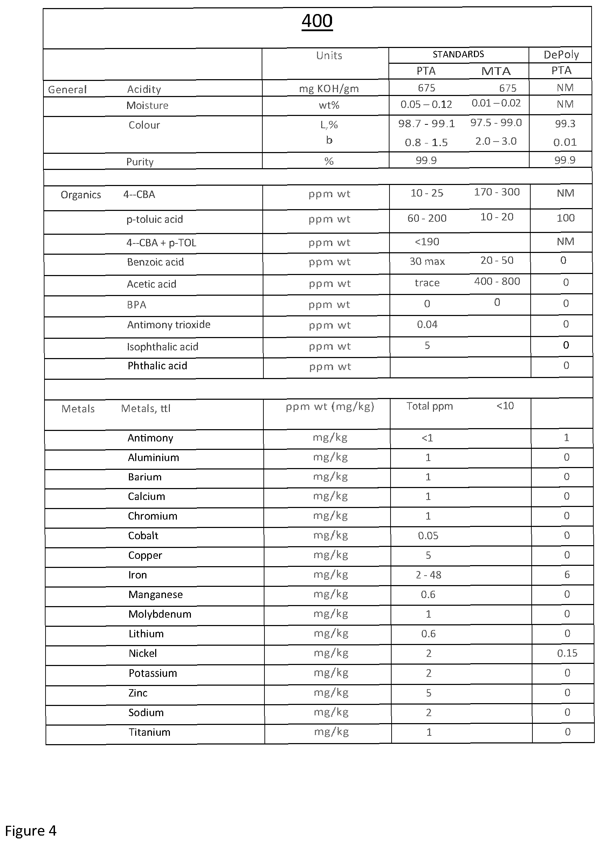

Referring now to Figure 4, shown therein is a table 400 detailing specifications of a purified terephthalic acid produced using method 100 described herein. Specifications detailed in table 400 include the concentration of contaminants in a sample of purified terephthalic acid. The sample may be compared to the listed standards, as shown in Figure 4.

## Claims

1. A method of purifying terephthalic acid, the method comprising:
contacting unpurified terephthalic acid with graphite, activated carbon, and molecular sieve to provide a reaction mixture;
stirring the reaction mixture for a first certain period of time;
filtering the reaction mixture, to provide a reaction mixture filtrate;
providing graphite, activated carbon, and molecular sieve to the reaction mixture filtrate;
stirring the reaction mixture filtrate for a second certain period of time;
filtering the reaction mixture filtrate, to provide a reaction output solution; and
precipitating purified terephthalic acid from the reaction output solution.

2. The method of claim 1, wherein the reaction mixture is stirred at a pH of 14.

3. The method of claim 1 or 2, wherein the reaction mixture filtrate is stirred at a pH of 7.

4. The method of any one of claims 1 to 3, wherein graphite, activated carbon, and molecular sieve are provided for contacting with the unpurified terephthalic acid at a 1:6:2 ratio.

5. The method of any one of claims 1 to 4, wherein graphite, activated carbon, and molecular sieve are provided to the reaction mixture filtrate at a 1:6:2 ratio.

6. The method of any one of claims 1 to 4, wherein purified terephthalic acid is precipitated from the reaction output solution using an acid.

7. The method of claim 6, wherein the acid is hydrochloric acid or sulfuric acid.

8. The method of any one of claims 1 to 7, wherein the first period of time is between 10 and 120 minutes.

9. The method of claim 6, wherein the first period of time is 30 minutes.

10. The method of any one of claims 1 to 9, wherein the second period of time is between 10 and 120 minutes.

11. The method of claim 10, wherein the second period of time is 30 minutes.

12. The method of any one of claims 1 to 11, wherein the method is performed at room temperature.

13. The method of any one of claims 1 to 12, wherein the molecular sieve contacted with the unpurified terephthalic acid is Zeolite 13X.

14. The method of any one of claims 1 to 13, wherein the molecular sieve provided to the reaction mixture filtrate is Zeolite 13X.

## Patentansprüche

1. Verfahren zum Reinigen von Terephthalsäure, wobei das Verfahren umfasst:
Inkontaktbringen von ungereinigter Terephthalsäure mit Graphit, Aktivkohle und Molekularsieb, um ein Reaktionsgemisch bereitzustellen;
Rühren des Reaktionsgemischs für einen ersten bestimmten Zeitraum;
Filtrieren des Reaktionsgemischs, um ein Reaktionsgemischfiltrat bereitzustellen;
Zugeben von Graphit, Aktivkohle und Molekularsieb zu dem Reaktionsgemischfiltrat;
Rühren des Reaktionsgemischfiltrats für einen zweiten bestimmten Zeitraum;
Filtrieren des Reaktionsgemischfiltrats, um eine Reaktionsausgangslösung bereitzustellen; und
Ausfällen von gereinigter Terephthalsäure aus der Reaktionsausgangslösung.

2. Verfahren nach Anspruch 1, wobei das Reaktionsgemisch bei einem pH-Wert von 14 gerührt wird.

3. Verfahren nach Anspruch 1 oder 2, wobei das Reaktionsgemischfiltrat bei einem pH-Wert von 7 gerührt wird.

4. Verfahren nach einem der Ansprüche 1 bis 3, wobei Graphit, Aktivkohle und Molekularsieb zum Inkontaktbringen mit der ungereinigten Terephthalsäure in einem Verhältnis von 1:6:2 bereitgestellt werden.

5. Verfahren nach einem der Ansprüche 1 bis 4, wobei Graphit, Aktivkohle und Molekularsieb dem Reaktionsgemischfiltrat in einem Verhältnis von 1:6:2 zugegeben werden.

6. Verfahren nach einem der Ansprüche 1 bis 4, wobei gereinigte Terephthalsäure nter Verwendung einer Säure aus der Reaktionsausgangslösung ausgefällt wird.

7. Verfahren nach Anspruch 6, wobei die Säure Salzsäure oder Schwefelsäure ist.

8. Verfahren nach einem der Ansprüche 1 bis 7, wobei der erste Zeitraum zwischen 10 und 120 Minuten beträgt.

9. Verfahren nach Anspruch 6, wobei der erste Zeitraum 30 Minuten beträgt.

10. Verfahren nach einem der Ansprüche 1 bis 9, wobei der zweite Zeitraum zwischen 10 und 120 Minuten beträgt.

11. Verfahren nach Anspruch 10, wobei der zweite Zeitraum 30 Minuten beträgt.

12. Verfahren nach einem der Ansprüche 1 bis 11, wobei das Verfahren bei Raumtemperatur durchgeführt wird.

13. Verfahren nach einem der Ansprüche 1 bis 12, wobei das mit der ungereinigten Terephthalsäure in Kontakt gebrachte Molekularsieb Zeolith 13X ist.

14. Verfahren nach einem der Ansprüche 1 bis 13, wobei das dem Reaktionsgemischfiltrat zugegebene Molekularsieb Zeolith 13X ist.

## Revendications

1. Procédé de purification d'acide téréphtalique, le procédé comprenant :
la mise en contact d'acide téréphtalique non purifié avec du graphite, du charbon actif et un tamis moléculaire afin de fournir un mélange réactionnel ;
l'agitation du mélange réactionnel pendant une première période de temps déterminée ;
la filtration du mélange réactionnel, afin de fournir un filtrat de mélange réactionnel ;
l'ajout de graphite, de charbon actif et d'un tamis moléculaire au filtrat de mélange réactionnel ;
l'agitation du filtrat de mélange réactionnel pendant une seconde période de temps déterminée ;
la filtration du filtrat de mélange réactionnel, afin de fournir une solution de sortie de réaction ; et
la précipitation d'acide téréphtalique purifié à partir de la solution de sortie de réaction.

2. Procédé selon la revendication 1, dans lequel le mélange réactionnel est agité à un pH de 14.

3. Procédé selon la revendication 1 ou 2, dans lequel le filtrat de mélange réactionnel est agité à un pH de 7.

4. Procédé selon l'une quelconque des revendications 1 à 3, dans lequel le graphite, le charbon actif et le tamis moléculaire sont fournis pour la mise en contact avec l'acide téréphtalique non purifié dans un rapport de 1:6:2.

5. Procédé selon l'une quelconque des revendications 1 à 4, dans lequel le graphite, le charbon actif et le tamis moléculaire sont ajoutés au filtrat de mélange réactionnel dans un rapport de 1:6:2.

6. Procédé selon l'une quelconque des revendications 1 à 4, dans lequel l'acide téréphtalique purifié est précipité à partir de la solution de sortie de réaction à l'aide d'un acide.

7. Procédé selon la revendication 6, dans lequel l'acide est de l'acide chlorhydrique ou de l'acide sulfurique.

8. Procédé selon l'une quelconque des revendications 1 à 7, dans lequel la première période de temps est comprise entre 10 et 120 minutes.

9. Procédé selon la revendication 6, dans lequel la première période de temps est de 30 minutes.

10. Procédé selon l'une quelconque des revendications 1 à 9, dans lequel la seconde période de temps est comprise entre 10 et 120 minutes.

11. Procédé selon la revendication 10, dans lequel la seconde période de temps est de 30 minutes.

12. Procédé selon l'une quelconque des revendications 1 à 11, dans lequel le procédé est mis en œuvre à température ambiante.

13. Procédé selon l'une quelconque des revendications 1 à 12, dans lequel le tamis moléculaire mis en contact avec l'acide téréphtalique non purifié est la zéolithe 13X.

14. Procédé selon l'une quelconque des revendications 1 à 13, dans lequel le tamis moléculaire ajouté au filtrat de mélange réactionnel est la zéolithe 13X.
